# EUROPÄISCHE PATENTSCHRIFT

(11) **EP 0 424 706 B1**
(45) Veröffentlichungstag und Bekanntmachung des Hinweises auf die Patenterteilung: **24.03.1993**
(21) Anmeldenummer: 90119086.8
(22) Anmeldetag: 05.10.1990
(51) Int. Cl.: A61K 31/60, A61K 9/20, A61K 9/68, A61K 9/46

(54) **Kaubare oder lutschbare Arzneiform, Verfahren zu ihrer Herstellung und ihre Verwendung**
Chewable or suckable pharmaceutical form, process for its preparation and its use
Forme galénique à mâcher ou à sucer, procédé pour sa préparation et son utilisation

(30) Priorität: 25.10.1989 DE 3935550
(43) Veröffentlichungstag der Anmeldung: 02.05.1991
(73) Patentinhaber: PHARMATRANS SANAQ AG, 4011 Basel (CH)
(72) Erfinder: Bauer, K.,Prof.Dr.Chem., W-7800 Freiburg-Tiengen (DE); Brune, K.,Prof.Dr.Chem., W-8525 Marloffstein (DE); Huber, Anton Sebastian, CH-4052 Basel (CH)
(74) Vertreter: Kraus, Walter, Dr.

(56) Entgegenhaltungen:
- EP-A- 0 153 836
- FR-A- 2 382 893
- US-A- 3 485 920
- US-A- 4 167 558
- US-A- 4 639 368
- US-A- 4 866 046

## Beschreibung

Die Erfindung betrifft eine kaubare oder lutschbare Arzneiform, die Acetylsalicylsäure enthält und die eine verbesserte Schleimhautverträglichkeit aufweist, sowie ein Verfahren zu ihrer Herstellung und ihre Verwendung.

Acetylsalicylsäure (ASS) wird seit mehreren Jahrzehnten als Analgetikum und Antirheumatikum therapeutisch verwendet. Neuerdings findet sie auch weitverbreitet als Thrombocyten-Aggregationshemmer (TAH) zur Prophylaxe und Behandlung von Thrombosen und Infarkten Verwendung.

Die Dosierungen von marktüblichen Acetylsalicylsäurezubereitungen liegen in der Regel zwischen 50 und 1000 mg pro Tablette.

Im Gegensatz zur gelegentlichen Einnahme als Analgetikum ist die Anwendung als Thrombocyten-Aggregationshemmer in der Regel eine Dauerapplikation, bei der eine gute Schleimhautverträglichkeit eine wichtige Rolle spielt. Acetylsalicylsäure ist bekanntlich relativ stark schleimhautreizend und verursacht in bestimmten Fällen sogar Magen- oder Darmblutungen, die sich durch Blut im Stuhl bemerkbar machen. Eine verbesserte Schleimhautverträglichkeit wäre daher insbesondere für Arzneimittel zur Prophylaxe und Behandlung von Thrombosen und Infarkten ein erheblicher Vorteil.

Eine Verbesserung der Schleimhautverträglichkeit hat man schon durch Mikroverkapselung der Acetylsalicylsäure versucht sowie durch den Zusatz von Puffern. Diese Verfahren sind jedoch für Arzneiformen, die gekaut oder gelutscht werden sollen, nicht ausreichend oder gar störend.

Die bekannten Acetylsalicylsäurezubereitungen besitzen weiterhin den Nachteil, daß ihre orale Resorption schlecht ist und somit nicht der erwünschte schnelle Wirkungseintritt möglich wird.

In der US-A-3485920 werden Arzneiformen, bestehend aus Acetylsalicylsäure, Pektin und anderen Hilfsstoffen beschrieben, die als Analgetikum eingesetzt werden. Durch die Hilfsstoffkombination konnte eine Geschmacksverbesserung und durch eine evtl. Zugabe von Natrium-Bikarbonat eine Verbesserung der Lösungsgeschwindigkeit erzielt werden. Als Beispiel werden u.a. Pastillen genannt, die aber Maissirup, Zuckersirup oder Honig enthalten. Eine bessere gastrointestinale Verträglichkeit soll durch die Herstellung einer "sustained release"-Arzneiform erreicht werden. Im Gegensatz dazu betrifft die erfindungsgemäße Zusammensetzung ein Präparat mit der Indikation "Hemmung der Aggregation von Blutplättchen". Diese Indikation wird in US-A-3485920 nicht beansprucht. Sie kann auch gar nicht beansprucht werden, weil die vorgeschlagenen Präparationen (z.B. Pastille) notgedrungen zu einer Hydrolyse der darin enthaltenen Acetylsalicylsäure führen müssen, da alle diese Arzneiformen in direkter oder indirekter Weise Wasser enthalten. Um eine bessere Magenverträglichkeit zu erreichen, wird in US-A-3485920 eine "sustained release"-Formulierung genannt. Die in der vorliegenden Anmeldung beanspruchten Arzneiformen zeigen diese bessere Magenverträglichkeit nicht durch Herstellung einer "sustained release"-Arzneiform, sondern durch eine spezielle Vorrezeptur. Die erfindungsgemäße Formulierung setzt den Wirkstoff auf überraschend schnelle Weise frei. Dies konnte überraschenderweise nur dadurch erreicht werden, daß beim Kau- oder Lutschprozeß Kohlendioxidgas entwickelt wird. Das Kohlendioxidgas bewirkt eine Schaumbildung, und diese bewirkt eine schnelle Freisetzung und Aufnahme des Wirkstoffs sowie eine bessere Verträglichkeit.

In FR-A-2382893 werden Arzneistoffzubereitungen beschrieben, die Cholestyramin enthalten. Diese Formulierungen sollen bei Patienten, die an duodenogastralen biliären Reflux leiden, eingesetzt werden. Cholestyramin, ein basisches Anionenaustauschharzgemisch, bindet körpereigene Gallensäuren und kann somit bei solchen Patienten ulkusprotektiv wirken. Im Gegensatz zu Acetylsalicylsäure besitzt der Arzneistoff Cholestyramin selbst keine mukosaschädigenden Eigenschaften. Durch den Zusatz von Alginsäure wird eine Viskositätserhöhung erzielt. Natrium-Bikarbonat wird eingesetzt, um die Alginsäure zu neutralisieren. Bei Auflösung der Arzneiform entsteht eine schäumende Masse, die einen Rückfluß verhindern kann. Dadurch ist es möglich, daß bei den in der FR-A-2382893 genannten Beispielen die ulkusprotektive Wirkung nicht nur durch Cholestyramin, sondern auch durch die Arzneistoffzubereitung gewährleistet wird.

In der EP-A-153836 werden Arzneiformen beschrieben, die bei Wasserkontakt einen Schaum mit einer verbesserten "Stabilität" bilden (Schaum als Arzneistoffcarrier). Als Beispiele werden verschiedene Pharmaka aufgeführt, die als Tabletten, Kapseln oder Granulat appliziert werden können. Ziel ist bei p.o. Applikation eine Bildung eines gastrointestinalen Depots, welches dann als "sustained release"-Form dienen soll.

Durch dieses Prinzip wird weder eine verbesserte Magenverträglichkeit noch eine schnelle Freisetzung beansprucht, und es wird im Gegenteil eine "sustained release" beschrieben.

In der US-A-4167558 wird eine neuartige "sustained release"Formulierung beschrieben. Durch die neuartige Hilfsstoffkombination wird erreicht, daß sich bei Kontakt mit Magensaft die Dichte der Formulierung ändert und diese somit "im Magensaft schwimmt". Diese "free-floating tablet" stellt das "sustained release"-Prinzip dar.

Der vorliegenden Erfindung liegt die Aufgabe zugrunde, eine Acetylsalicylsäurezubereitung mit verbesserter Schleimhautverträglichkeit zur Verfügung zu stellen. Die erfindungsgemäße Zubereitung soll insbesondere für eine Dauerapplikation geeignet sein und zur Prophylaxe und Behandlung von Thrombosen und Infarkten Verwendung finden. Die erfindungsgemäße Zubereitung soll eine bessere orale Resorption ermöglichen, als dies mit den bekannten Acetylsalicylsäure enthaltenden Arzneimitteln möglich ist.

Es wurde nun gefunden, daß die Verträglichkeit der Acetylsalicylsäure besonders gut ist, wenn sie als kau- oder lutschbare Zubereitung, zum Beispiel als Kau- oder Lutschtablette, Kaugummi oder Kaugranulat, verabreicht wird, so daß eine längere Aufenthaltszeit in der Mundhöhle herbeigeführt wird und sich dabei ein schleimartiger Schaum entwickelt, der die Acetylsalicylsäurekristalle einhüllt. Die Formulierung solcher Zubereitungen muß deshalb derart aufgebaut sein, daß sich beim Kau- bzw. Lutschprozeß und beim Vermischen der Bestandteile der zerkauten Zubereitung mit Speichel infolge Reaktion einer sauren Komponente mit einem CO₂-Entwickler CO₂-Gas entwickeln kann. Überraschenderweise wurde weiterhin gefunden, daß schleimbildende Proteine und/oder Polysaccharide in der Formulierung dieser Applikationsformen enthalten sein müssen und daß es wichtig ist, daß beim Zerkauen die Schleimbildner und die CO₂ entwickelnde Kombination die in die Formulierung eingearbeiteten therapeutisch wirksamen Acetylsalicylsäurekristalle einzeln mit einem feinblasigen Schleimschaum einhüllen.

Gegenstand der Erfindung ist eine kaubare
oder lutschbare Arzneiform, dadurch **gekennzeichnet,** daß sie
- 100: Gew.-Teile Acetylsalicylsäure,
- 25 - 80: Gew.-Teile Zitronensäure, Weinsäure, primäres Natriumcitrat, saures Natrium- oder Kaliumsulfat, Adipinsäure oder deren Gemische,
- 20 - 1000: Gew.-Teile Pektine, Alginsäuren, Galactomane, Tragant oder deren Gemische,
- 15 - 60: Gew.-Teile Natrium-, Kalium-, Calcium-, Magnesiumcarbonat oder -bicarbonat oder deren Gemische,
- 30 - 2000: Gew.-Teile Gelatine, hydrolysierte Gelatine, Casein, Hühnereiweiß oder anderes Tiereiweiß oder deren Gemische und

(a) Zucker, Zuckeralkohole, Süßmittel, Aromen,
(b) sonstige übliche Granulier- und/oder Tablettierungs-Hilfsstoffe und/oder
(c) Kaugummi-Grundmassen

jeweils, soweit erforderlich, enthält,
wobei die feste Arzneiform nach Aufschwemmung in Wasser im Verhältnis 1:10 einen pH-Wert im Verhältnis von 2 bis 6, vorzugsweise 2 bis 4, aufweist.

Die Erfindung betrifft weiterhin ein Verfahren zur Herstellung der oben genannten kaubaren oder lutschbaren Arzneiform, das dadurch gekennzeichnet ist, daß 100 Gew.Teile feingemahlene Acetylsalicylsäure (ASS) intensiv mit
- 20 - 1000: Gew.-Teilen des Polysaccharids und
- 30 - 2000: Gew.-Teilen des Proteins vermischt werden,
- 25 - 80: Gew.-Teile der genießbaren Säure oder eines sauren Salzes einer solchen Säure,
- 15 - 60: Gew.-Teile des genießbaren Carbonats oder Bicarbonats und

(a) Zucker, Zuckeralkohole, Süßmittel, Aromen und
(b) sonstige übliche Granulier- und/oder Tablettierungs-Hilfsstoffe und/oder
(c) Kaugummi-Grundmassen

dem Gemisch beigemischt werden,
wobei die gesamte genannte Säure oder ihr Salz oder ein Teil davon gegebenenfalls auch gleich mit der Acetylsalicylsäure und dem Polysaccharid und/oder dem Protein vermischt werden kann.

Die Erfindung betrifft weiterhin die oben genannte kaubare oder lutschbare Arzneiform zur Verwendung als Thrombocyten-Aggregationshemmer zur Prophylaxe und Behandlung von Thrombosen und Infarkten.

Durch lutschbare oder kaubare Applikationsformen werden zweierlei vorteilhafte Effekte erzielt. Erstens wird eine bessere orale, zum Beispiel sublinguale oder bukkale, Resorption erreicht, die einen rascheren Wirkungseintritt ermöglicht, und zweitens wird durch die Einhüllung der einzelnen ASS-Kristalle in den Schleimschaum direkte Wechselwirkung von ASS mit Magen- und Darmschleimhäuten eingeschränkt oder in den günstigsten Fällen verhindert. Auf diese Weise kommt die überraschend gute Schleimhautverträglichkeit dieser Zubereitungen zustande.

Die Spannen für das Polysaccharid und das Pektin sind relativ hoch. Der Grund dafür ist, daß die Verträglichkeit umso besser wird, je mehr man zusetzt. Grenzen ergeben sich durch eine Verschlechterung der Bioverfügbarkeit mit der Erhöhung des Zusatzes sowie durch die Größe der entstehenden Arzneiform. Bei Tabletten ist das entstehende Volumen bzw. die Größe streng limitierend, bei Granulaten erheblich weniger.

Die erfindungsgemäße Zubereitung muß weiterhin 15 bis 60, vorzugsweise 15 bis 20, Gew.-Teile Natrium-, Kalium-, Calcium-, Magnesiumcarbonat oder -bicarbonat oder deren Gemische enthalten.

Die erfindungsgemäße Zubereitung enthält Zucker oder Zuckeralkohole, Süßmittel, Aromen und sonstige Granulier- und/oder Tablettier-Hilfsstoffe oder Kaugummi-Grundmassen jeweils in Mengen wie es erforderlich ist. Die erforderlichen Mengen können vom Fachmann leicht anhand von Vorversuchen bestimmt werden. Beispiele für Zucker und Zuckeralkohole sind Saccharose, Glucose, Lactose, Mannitol, Sorbitol oder deren Gemische. Beispiele für Süßmittel sind Saccharin-Natrium, Natrium-Cyclamat, Aspartam, Acesulfam oder deren Gemische, und Beispiele für Aromen sind Citrusaroma, Orangenaroma, Grapefruitaroma, Johannisbeeraroma, Pfefferminzarome oder deren Gemische.

Wenn die Arzneizubereitungen in Form von Kautabletten vorliegen, müssen sie zwingend die dafür notwendigen und in der allgemeinen Vorschrift genannten Kautabletten-Grundmassen, Hilfsstoffe usw. enthalten. Für Kaugummi ist eine Kaumasse zwingend. Die Herstellung von Lutschtabletten ist dem Fachmann ebenfalls geläufig. Zur Tablettenkomprimierung sind Schmier- und Formtrennmittel in der Regel zwingend.

Kautabletten werden wie normale Tabletten hergestellt, sie dürfen jedoch keine Sprengmittel oder Zerfallsbeschleuniger enthalten. Die Kautabletten müssen so weich verpreßt werden, daß sie kaubar sind.

Lutschtabletten dürfen ebenfalls keine Sprengmittel oder Zerfallsbeschleuniger enthalten. Sie zeichnen sich durch verhältnismäßig hohe Gehalte an gut löslichen Zuckern oder Zuckeralkoholen aus, zum Beispiel Saccharose oder Mannitol oder Sorbitol. Die Lutschtabletten werden sehr hart gepreßt, daß gelutscht, aber nicht gekaut werden kann.

Um die vorgesehenen Reaktionen, die Schleimschaumentwicklung und die Einhüllung der ASS-Kristalle in diesen Schleimschaum, sicher zu erreichen, muß bereits bei der Herstellung dieser Zubereitungen in bestimmter, nachfolgend beschriebener Weise verfahren werden. Bei der Herstellung der Granulate oder Tablettiermischungen werden zunächst die feingemahlenen ASS-Kristalle intensiv mit den Schleimbildnern vermischt bzw. in diese eingebettet. Anschließend werden die restlichen Bestandteile der Formulierung wie üblich eingearbeitet.

Damit die relativ hydrolyseempfindliche ASS weder bei der Befeuchtung, zum Beispiel bei der Feuchtgranulation, noch bei der späteren Lagerung, zum Beispiel durch feuchte Atmosphäre, mehr als vertretbar abgebaut wird, ist es sinnvoll, alle ASS-haltigen Vormischungen oder die Grundmasse jeder ASS-Zubereitung mit Säure auf einen pH um 2 bis 3 einzustellen. Bei dem pH-Wert 2, 3, der zweckmäßigerweise in einer 10%igen wäßrigen Lösung oder Aufschwemmung gemessen wird, zeigt ASS die langsamste bzw. niedrigste Hydrolysegeschwindigkeit.

Beim Zerkauen der erfindungsgemäßen Zubereitungen nach der peroralen Einnahme sollen die Schleimbildner zu quellen beginnen und etwa gleichzeitig soll das CO₂-Entwicklergemisch anfangen, CO₂-Gas zu bilden. Auf diese Weise soll durch das Zusammenwirken dieser beiden Reaktionen ein schleimiger Schaum entstehen, der die einzelnen ASS-Kristalle feindispers einhüllt. Der schleimige Schaum soll den direkten Kontakt von ASS mit den Schleimhäuten des Verdauungstraktes so weit wie möglich abschwächen bzw. verhindern. Die ASS wird aus dem schleimigen Schaum durch in-Lösung-gehen nach und nach freigesetzt und kann dann sowohl durch sublinguale und/oder bukkale Resorption aus der Mundhöhle resorbiert werden oder auch nach dem Hinunterschlucken auch durch Permeation der Magen- oder Darmschleimhäute.

Die bessere Verträglichkeit der erfindungsgemäßen Zubereitungen wurde wie folgt geprüft.

Acht Probanden erhielten im blinden "Cross-over"-Versuch jeweils eine handelsübliche (aromatisierte) 100-mg-ASS-Tablette und zu einem anderen Zeitpunkt eine erfindungsgemäße 100-mg-ASS-Kautablette. Die Probanden wurden angehalten, die Tabletten, ohne sie zu zerkauen, 30 Minuten unter der Zunge zu halten und sich auflösen zu lassen. Jeweils nach 15 und 30 Minuten wurde von einem "blinden" Beobachter der Grad der Irritation der Schleimhaut festgestellt.

Überraschenderweise zeigte sich, daß bei den Probanden, die die erfindungsgemäße Zusammensetzung erhielten, keine Irritation, d.h. Rötung der Schleimhaut, festgestellt werden konnte. Die Probanden gaben ebenfalls an, daß sie kein unangenehmenes Gefühl im Mund verspürten.

Die folgenden Beispiele erläutern die Erfindung.

### Beispiel 1

100,0 kg Acetylsalicylsäure werden mit 50,0 kg Galactomannan und 35,0 kg Zitronensäure vermischt und anschließend mit einer Lösung von 15,0 kg Zitronensäure in ca. 75,0 kg wäßrigem 80%igen Alkohol angeknetet. Falls erforderlich, wird noch etwas mehr 80%iger Alkohol zugesetzt, bis die Masse ausreichend plastisch ist. Dann wird bei 60°C getrocknet und die getrocknete Mischung durch ein 1-mm-Sieb geschlagen.

In einem zweiten Teilansatz werden 30,0 kg Calciumcarbonat, 59,0 kg Mannitol und 10,0 kg hydrolysierte Gelatine mit einer ausreichenden Menge Wasser, in der vorher 0,5 kg Saccharin-Natrium gelöst wurden, feuchtgranuliert und ebenfalls durch ein 1-mm-Sieb geschlagen. Zuletzt werden die beiden Teilansätze gemischt, 0,4 kg Passionsfruchtaroma sowie 0,5 bis 1,0 kg pulverisiertes Paraffinwachs als Schmier- und Gleitmittel zugesetzt und in üblicher Weise zu Kautabletten mit einem Bruttogewicht von 300 mg pro Tablette komprimiert.

### Beispiel 2

50,0 kg Acetylsalicylsäure werden mit 100,0 kg Pektin und 15,0 kg Weinsäure intensiv gemischt und mit einer ausreichenden Menge 70%igem Alkohol angeknetet, bis eine genügend plastische Masse ansteht, anschließend wird durch ein grobes Sieb (5 mm lichte Maschenweite) geschlagen und bei 80°C getrocknet. Das getrocknete Granulat wird durch ein Sieb mit 0,8 mm lichter Maschenweite geschlagen.

Separat hiervon wird ein zweites Teilgranulat aus 100,0 kg Dextrose, 20,0 kg Calciumcarbonat und 9,5 kg hydrolysierter Gelatine mit einer ausreichenden Menge Wasser, in der 0,5 kg Süßmittel gelöst wurden, hergestellt. Nach der Zugabe von 0,3 kg Orangenaroma und 0,5 kg Magnesiumstearat werden in üblicher Weise Lutschtabletten mit einem Bruttogewicht von 300 mg pro Tablette gepreßt.

### Beispiel 3

100,0 kg Acetylsalicylsäure werden mit 30,0 kg Zitronensäure und 200,0 kg Galactomannan gut gemischt und mit 70%igem wäßrigen Methanol zu einer plastischen Masse angeknetet, durch ein Sieb mit 5 mm lichter Maschenweite feuchtgranuliert und mit Umluft von 80°C getrocknet. Das getrocknete Granulat wird durch ein 1-mm-Sieb geschlagen. In einem zweiten Teilansatz werden 1570,0 kg Mannitol, 50,0 kg Natriumcarbonat und 40,0 kg hydrolysierte Gelatine mit einer ausreichenden Menge Wasser, in der vorher 1,0 kg Natriumcyclamat/Natriumsaccharinat, 9:1, gelöst wurden, feuchtgranuliert, gesiebt und getrocknet. Die beiden Teilansätze werden vermischt, mit dem Aroma (zum Beispiel Zitronenaroma), Schmier- und Gleitmitteln versetzt und in üblicher Weise zu Kautabletten mit einem Bruttogewicht von 2000 mg verpreßt.

### Beispiel 4

### Allgemeine Vorschrift zur Herstellung der erfindungsgemäßen Kaugummi

Erfindungsgemäße Kaugummi werden hergestellt, indem zunächst 100 Gew.-Teile feinpulverisierte Acetylsalicylsäurekri stalle mit 10 bis 100 Gew.-Teilen Säure, wie Zitronensäure, 0 bis 5000 Gew.-Teilen Polysaccharid und 0 bis 5000 Gew.-Teilen Protein mit wäßrig-alkoholischer Lösung angeknetet werden.

Diese plastische Masse wird dann nach Abtrocknen der Alkohol-Wasser-Mischung in eine ausreichende Menge einer üblichen Kaugummi-Grundlage, zum Beispiel aus 20% Chicle, Butylkautschuk, Gutta, PVAc-Homo- oder -Copolymeren oder ähnlichen, ca. 60% Saccharose, 20% Glucosesirup, 0,5% Glycerol, 0,5% Süßmittel und 0,1 bis 1% Aromastoffen eingearbeitet. Die Kaugummi-Grundlage wird außerdem noch mit 10 bis 100 Gew.-Teilen eines genießbaren Carbonats oder Bicarbonats, bezogen auf 100 Gew.-Teile Acetylsalicylsäure, versetzt. Anschließend werden die Kaugummi geformt und verpackt.

## Patentansprüche

1. Kaubare oder lutschbare Arzneiform, dadurch **gekennzeichnet,** daß sie
100 Gew.-Teile Acetylsalicylsäure,
25 - 80 Gew.-Teile Zitronensäure, Weinsäure, primäres Natriumcitrat, saures Natrium- oder Kaliumsulfat, Adipinsäure oder deren Gemische,
20 - 1000 Gew.-Teile Pektine, Alginsäuren, Galactomane, Tragant oder deren Gemische,
15 - 60 Gew.-Teile Natrium-, Kalium-, Calcium-, Magnesiumcarbonat oder -bicarbonat oder deren Gemische,
30 - 2000 Gew.-Teile Gelatine, hydrolysierte Gelatine, Casein, Hühnereiweiß oder anderes Tiereiweiß oder deren Gemische und
(a) Zucker, Zuckeralkohole, Süßmittel, Aromen,
(b) sonstige übliche Granulier- und/oder Tablettierungs-Hilfsstoffe und/oder
(c) Kaugummi-Grundmassen
jeweils, soweit erforderlich, enthält,
wobei die feste Arzneiform nach Aufschwemmung in Wasser im Verhältnis 1:10 einen pH-Wert im Verhältnis von 2 bis 6, vorzugsweise 2 bis 4, aufweist.

2. Kaubare oder lutschbare Arzneiform nach Anspruch 1, dadurch **gekennzeichnet,** daß sie als Zucker und Zuckeralkohole Saccharose, Glucose, Lactose, Mannitol, Sorbitol oder deren Gemische, als Süßmittel Saccharin-Natrium, Natrium-Cyclamat, Aspartam, Acesulfam oder deren Gemische und als Aromen Zitrusaroma, Orangenaroma, Grapefruitaroma, Johannisbeeraroma, Pfefferminzaroma oder deren Gemische enthält.

3. Kaubare oder lutschbare Arzneiform nach Anspruch 1 oder 2, dadurch **gekennzeichnet,** daß sie in Form von Kautabletten, Lutschtabletten oder als Kaugummi vorliegt.

4. Kaubare oder lutschbare Arzneiform nach Anspruch 1 oder 2, dadurch **gekennzeichnet,** daß sie in Form zuckerfreier Kautabletten, Lutschtabletten oder als zuckerfreies Kaugummi vorliegt.

5. Verfahren zur Herstellung der kaubaren oder lutschbaren Arzneiform nach mindestens einem der Ansprüche 1 bis 4, dadurch **gekennzeichnet,** daß 100 Gew.Teile feingemahlene Acetylsalicylsäure (ASS) intensiv mit
20 - 1000 Gew.-Teilen des Polysaccharids und
30 - 2000 Gew.-Teilen des Proteins vermischt werden,
25 - 80 Gew.-Teile der genießbaren Säure oder eines sauren Salzes einer solchen Säure,
15 - 60 Gew.-Teile des genießbaren Carbonats oder Bicarbonats und
(a) Zucker, Zuckeralkohole, Süßmittel, Aromen und
(b) sonstige übliche Granulier- und/oder Tablettierungs-Hilfsstoffe und/oder
(c) Kaugummi-Grundmassen
dem Gemisch beigemischt werden,
wobei die gesamte genannte Säure oder ihr Salz oder ein Teil davon gegebenenfalls auch gleich mit der Acetylsalicylsäure und dem Polysaccharid und/oder dem Protein vermischt werden kann.

6. Verfahren nach Anspruch 5, dadurch **gekennzeichnet,** daß die Vermischung der genannten Bestandteile auf solche Weise erfolgt, daß alle Acetylsalicylsäure enthaltenden Vormischungen bzw. Grundmassen einen pH-Wert im Bereich von 2 bis 4 aufweisen, wobei zur Einstellung des pH-Werts ein Teil oder die gesamte genießbare Säure oder deren Säuresalz verwendet wird.

7. Kaubare oder lutschbare Arzneiform nach mindestens einem der Ansprüche 1 bis 5 zur Verwendung als Thrombocyten -Aggregationshemmer zur Prophylaxe und Behandlung von Thrombosen und Infarkten.

## Claims

1. A medicinal formulation for chewing or sucking, characterized in that it contains
100 parts by weight acetyl salicylic acid,
25 to 80 parts by weight citric acid, tartaric acid, primary sodium citrate, acidic sodium or potassium sulfate, adipic acid or mixtures thereof,
20 to 1,000 parts by weight pectins, alginic acids, galactomannans, tragacanth or mixtures thereof,
15 to 60 parts by weight sodium, potassium, calcium, magnesium carbonate or bicarbonate or mixtures thereof,
30 to 2,000 parts by weight gelatine, hydrolyzed gelatine, casein, chicken protein or other animal protein or mixtures thereof
and, where necessary,
(a) sugar, sugar alcohols, sweeteners, flavourings,
(b) other typical granulation and/or tabletting aids and/or
(c) chewing gum bases,
the solid medicinal formulation having a pH value of 2 to 6 and preferably 2 to 4 after suspension in water in a ratio of 1:10.

2. A medicinal formulation for chewing or sucking as claimed in claim 1, characterized in that it contains sucrose, glucose, lactose, mannitol, sorbitol or mixtures thereof as sugars and sugar alcohols, saccharin sodium, sodium cyclamate, aspartame, acesulfam or mixtures thereof as sweeteners and citrus flavouring, orange flavouring, grapefruit flavouring, red currant flavouring, peppermint flavouring or mixtures thereof as the flavouring.

3. A medicinal formulation for chewing or sucking as claimed in claim 1 or 2, characterized in that it is present in the form of chewing tablets, sucking tablets or chewing gum.

4. A medicinal formulation for chewing or sucking as claimed in claim 1 or 2, characterized in that it is present in the form of sugar-free chewing tablets, sucking tablets or sugar-free chewing gum.

5. A process for the production of the medicinal formulation for chewing or sucking claimed in at least one of claims 1 to 4, characterized in that 100 parts by weight finely ground acetyl salicylic acid (ASS) are intensively mixed with
20 to 1,000 parts by weight of the polysaccharide and
30 to 2,000 parts by weight of the protein; and
25 to 80 parts by weight of the edible acid or an acidic salt of such an acid,
15 to 60 parts by weight of the edible carbonate or bicarbonate and
(a) sugars, sugar alcohols, sweeteners, flavourings and
(b) other typical granulation and/or tabletting aids and/or
(c) chewing gum bases
are added to the mixture; all or part of the acid mentioned or its salt may also be directly mixed with the acetyl salicylic acid and the polysaccharide and/or the protein.

6. A process as claimed in claim 5, characterized in that the constituents mentioned are mixed in such a way that all the premixes or bases containing acetyl salicylic acid have a pH value in the range from 2 to 4, all or part of the edible acid or its acid salt being used to adjust the pH value.

7. A medicinal formulation for chewing or sucking as claimed in at least one of claims 1 to 5 for use as a thrombocyte aggregation inhibitor for the prophylaxis and treatment of thromboses and infarcts.

## Revendications

1. Forme galénique à mâcher ou à sucer, caractérisée en ce qu'elle contient à chaque fois, si cela est nécessaire :
100 parties en poids d'acide acétylsalicylique,
25 - 80 parties en poids d'acide citrique, d'acide tartrique, de citrate de sodium primaire, de sulfate acide de sodium ou de potassium, d'acide adipique, ou de mélanges de ces substances,
20 - 1000 parties en poids de pectines, d'acides alginiques, de galactomannanes, de gomme adragante, ou de mélanges de ces substances,
15 - 60 parties en poids de carbonate ou de bicarbonate de sodium, de potassium, de calcium ou de magnésium, ou de mélanges de ces substances,
30 - 2000 parties en poids de gélatine, de gélatine hydrolysée, de caséine, de blanc d'oeuf de poule ou de blanc d'oeuf d'autres animaux, ou de mélanges de ces substances; et
(a) des sucres, des alcools de sucre, des édulcorants et des arômes,
(b) d'autres adjuvants usuels pour la fabrication de granulés ou de comprimés, et/ou
(c) des compositions de base pour pâtes à mâcher, la forme galénique solide, après une mise en suspension dans de l'eau selon un rapport de 1:10, étant à pH 2 à 6, de préférence de 2 à 4.

2. Forme galénique à mâcher ou à sucer selon la revendication 1, caractérisée en ce qu'elle contient, comme sucres et alcools de sucres, du saccharose, du glucose, du lactose, du mannitol, du sorbitol, ou des mélanges de ces substances, comme édulcorants, de la saccharine sodique, du cyclamate de sodium, de l'aspartam, de l'acésulfame, ou des mélanges de ces substances, et comme arômes, l'arôme de citron, l'arôme d'orange, l'arôme de pamplemousse, l'arôme de groseille, l'arôme de menthe poivrée, ou des mélanges de ces substances.

3. Forme galénique à mâcher ou à sucer selon la revendication 1 ou 2, caractérisée en ce qu'elle se présente sous la forme de comprimés à mâcher, de comprimés à sucer ou de gomme à mâcher.

4. Forme galénique à mâcher ou à sucer selon la revendication 1 ou 2, caractérisée en ce qu'elle se présente sous la forme de comprimés à mâcher ou de comprimés à sucer ne contenant pas de sucre ou de gomme à mâcher ne contenant pas de sucre.

5. Procédé de préparation de la forme galénique à mâcher ou à sucer selon au moins une des revendications 1 à 4, caractérisé en ce qu'on mélange intimement 100 parties en poids d'acide acétylsalicylique (ASS) finement broyé avec
20 - 1000 parties en poids du polysaccharide et
30 - 2000 parties en poids de la protéine, et on ajoute à ce mélange :
25 - 80 parties en poids de l'acide comestible ou d'un sel acide d'un tel acide,
15 - 60 parties en poids du carbonate ou du bicarbonate comestible, et
(a) des sucres, des alcools de sucre, des édulcorants, des arômes, et
(b) d'autres adjuvants usuels pour la fabrication de granulés ou de comprimés, et/ou
(c) des compositions de base pour gommes à mâcher, la quantité totale dudit acide ou de son sel, ou une partie de celui-ci, pouvant éventuellement être mélangée simultanément avec l'acide acétylsalicylique et le polysaccharide et/ou la protéine.

6. Procédé selon la revendication 5, caractérisé en ce qu'on effectue le mélange desdits constituants de manière telle que l'ensemble des prémélanges et des compositions de base contenant l'acide acétylsalicylique ont un pH compris entre 2 et 4, et pour régler le pH, on utilise une partie ou la quantité totale de l'acide comestible ou de son sel.

7. Forme galénique à mâcher ou à sucer selon au moins une des revendications 1 à 5, pour une utilisation en tant qu'inhibiteur d'agrégation des thrombocytes pour la prophylaxie et le traitement des thromboses et des infarctus.
